# FASCICULE DE BREVET EUROPEEN

(11) **EP 4 032 574 B1**
(45) Date de publication et mention de la délivrance du brevet: **30.10.2024**
(21) Numéro de dépôt: 21214585.8
(22) Date de dépôt: 15.12.2021
(51) Int. Cl.: A61M 16/20, F16K 7/17, A61M 16/10

(54) **VENTILATEUR MÉDICAL À VALVE EXPIRATOIRE DOTÉE D'UN ÉLÉMENT DE VALVE DÉFORMABLE BI-MATIÈRE**
MEDIZINISCHES BEATMUNGSGERÄT MIT EINEM AUSATEMVENTIL MIT EINEM VERFORMBAREN VENTILELEMENT AUS ZWEI MATERIALIEN
MEDICAL FAN WITH EXPIRATORY VALVE WITH A BI-MATERIAL DEFORMABLE VALVE ELEMENT

(30) Priorité: 20.01.2021 FR 2100539
(43) Date de publication de la demande: 27.07.2022
(73) Titulaire: Air Liquide Medical Systems, 92182 Antony Cedex (FR); Air Liquide Medical Systems S.r.l., 20152 Milano (IT)
(72) Inventeur: MEREAUX, Beryl, 78350 Jouy en Josas (FR); BONAMARTE, Maximilien, 92182 Antony Cedex (FR); GALLONI, Bruno, 92182 Antony Cedex (FR); ALBERICI, Luca, 25073 Bovezzo (IT)
(74) Mandataire: Air Liquide

(56) Documents cités:
- EP-A2- 0 143 618
- FR-A1- 2 784 587
- US-A- 4 257 453

## Description

L'invention concerne l'utilisation d'une valve expiratoire comprenant un élément de valve déformable bi-matière au sein de la branche expiratoire d'un ventilateur médical servant à fournir un gaz respiratoire, tel de l'air ou de l'air enrichi en oxygène, à un patient.

Il est connu d'agencer dans la branche expiratoire du circuit de gaz d'un ventilateur médical, c'est-à-dire d'un appareil d'assistance respiratoire ou de ventilation assistée, une valve expiratoire servant à contrôler l'échappement du gaz expiré vers l'atmosphère et la pression expiratoire positive pendant les phases expiratoires des patients.

Une telle valve expiratoire comprend habituellement un corps de valve rigide traversé par un passage de gaz communiquant avec l'atmosphère ambiante, et un élément de valve déformable, c'est-à-dire flexible ou souple, comprenant un corps creux définissant un volume interne, qui coopèrent l'un avec l'autre pour contrôler l'échappement du gaz expiré vers l'atmosphère au travers de la valve expiratoire.

L'élément de valve déformable comprend généralement aussi un élément annulaire formant une collerette solidaire du corps creux et permettant de maintenir en position l'élément de valve déformable au sein du ventilateur.

Pour ce faire, l'élément de valve déformable (i.e. l'élément annulaire formant la collerette et le corps creux) est généralement formé d'une pièce en un matériau souple. FR-A-2784587 enseigne un élément de valve pour ventilateur de ce type.

Le corps creux définissant le volume interne de l'élément de valve déformable comprend une paroi souple formant une membrane flexible pouvant se déformer sous l'effet de la pression du gaz s'exerçant dans le volume interne de l'élément de valve déformable. En faisant varier la pression dans le volume interne de l'élément de valve déformable, la paroi souple formant une membrane flexible vient plus ou moins obturer le passage de gaz du corps de valve rigide, ce qui permet de fixer ou d'ajuster la pression expiratoire de la valve expiratoire, donc de contrôler l'échappement du gaz expiré par le patient (i.e. contenant du CO₂) vers l'atmosphère, pendant les phases expiratoires du patient. En faisant varier la pression s'exerçant dans le volume interne de la valve expiratoire, donc sur la paroi souple formant la membrane, on crée une résistance à l'expiration du patient, appelée Pression Expiratoire Positive ou PEP.

Par ailleurs, pendant les phases d'inspiration, la membrane flexible de la valve expiratoire obture totalement le passage de gaz du corps de valve rigide, donc la branche expiratoire, en créant une étanchéité fluidique avec le corps de valve, et donc empêcher au gaz de circuler au travers de la valve et d'être échappé vers l'atmosphère.

D'une façon générale, afin de pouvoir générer la PEP désirée, la paroi souple formant la membrane flexible de l'élément de valve déformable doit avoir une certaine course de déformation. Or, il est apparu en pratique que, du fait des contraintes d'assemblage (i.e. membrane dans la valve, valve dans le ventilateur...) et de celles liées au circuit patient comprenant la branche expiratoire sur laquelle est agencée la valve expiratoire, la paroi souple formant membrane flexible s'affaisse ou s'enfonce vers l'intérieur du corps de valve rigide, ce qui l'empêche alors de se pouvoir se déformer suffisamment pour laisser passer le gaz expiré, donc bloque l'expiration du patient.

Ce phénomène, appelé « frein expiratoire », est à éviter absolument car un patient souffrant de troubles respiratoires doit pouvoir évacuer facilement les gaz riches en CO₂ qu'il expire.

A l'inverse, on peut citer EP-A-0143618, US-A-4,257,453 qui enseignent un élément de valve déformable comprenant un corps central entouré d'une bordure périphérique annulaire, dans lequel la bordure est plus souple que le corps central. Toutefois, rendre le corps central plus rigide n'est pas souhaitable lorsque l'on souhaite générer une PEP donnée grâce à la flexibilité de la valve.

Le problème est dès lors de pouvoir résoudre le problème d'affaissement ou d'enfoncement de la valve expiratoire agencée sur la branche expiratoire du circuit de gaz d'un ventilateur médical, qui est utilisée pour contrôler la PEP lors des phases expiratoires du patient, c'est-à-dire d'améliorer les valves expiratoires connues.

La solution de l'invention concerne alors un ventilateur médical, c'est-à-dire un appareil d'assistance respiratoire, comprenant un circuit de gaz avec une branche expiratoire communiquant fluidiquement avec l'atmosphère et comprenant une valve expiratoire, ladite valve expiratoire comprenant un corps de valve rigide traversé par un passage de gaz et un élément de valve déformable agencé sur ledit passage de gaz de manière à contrôler la circulation de gaz dans ledit passage de gaz, ledit élément de valve déformable comprenant :
- un corps creux définissant un volume interne et comprenant une ouverture communiquant avec ledit volume interne, et
- une collerette annulaire solidaire du corps creux et agencée coaxialement autour de ladite ouverture.

Selon l'invention, le corps creux et la collerette annulaire sont formés d'un premier matériau souple, et un élément de rondelle formé d'un second matériau de rigidité supérieure au premier matériau est agencé sur la collerette annulaire.

Selon le mode de réalisation considéré, l'invention peut comprendre l'une ou plusieurs des caractéristiques suivantes :
- le premier matériau souple présente une première dureté Shore A (dsA1) et le second matériau présente une rigidité représentée par une seconde dureté Shore A (dsA2), avec dsA2 > dsA1.
- la première dureté Shore A (dsA1) inférieure ou égale à 50 shore A, de préférence inférieure ou égale à 40 shore A.
- le premier matériau est un élastomère de type silicone, de préférence du silicone biocompatible.
- le second matériau est un polymère.
- le second matériau est un polycarbonate (PC), un polyamide (PA) ou un polyacétal (POM), avantageusement le second matériau est un polyamide (PA) ou un polyacétal (POM).
- alternativement, le second matériau présente une seconde dureté (dsA2) d'au moins 80 shore A, de préférence le second matériau est un second type de silicone présentant une seconde dureté (dsA2) d'au moins 80 shore A.
- le corps creux et la collerette annulaire sont formés d'une pièce en le premier matériau souple.
- l'élément de rondelle est rigide.
- l'élément de rondelle est une rondelle, c'est-à-dire un disque mince avec un trou (i.e. une ouverture traversante) en son centre.
- l'élément de rondelle est agencé coaxialement sur la collerette annulaire.
- l'élément de rondelle est superposé à la collerette annulaire.
- l'élément de rondelle est préférentiellement agencé, i.e. posé, sur la collerette annulaire sans y être fixé.
- alternativement, l'élément de rondelle est agencé sur la collerette annulaire en y étant fixé, par exemple collé.
- l'élément de rondelle forme une couche rigide en polymère recouvrant la collerette annulaire.
- le corps creux et la collerette annulaire sont fixés l'un à l'autre par un col tubulaire agencé coaxialement à l'ouverture du corps creux.
- le corps creux, la collerette annulaire et le col tubulaire sont formés d'une seule pièce, par exemple par moulage par injection.
- le corps creux, le col et la collerette annulaire sont formés d'une seule pièce par moulage par injection.
- une ligne d'amenée de pression est reliée fluidiquement à l'ouverture du corps creux de l'élément de valve déformable de manière à fournir une pression gazeuse au volume interne du corps creux.
- le corps creux définissant le volume interne de l'élément de valve déformable comprend une paroi flexible venant au moins partiellement obturer le passage de gaz du corps de valve rigide de manière à contrôler ou ajuster le débit de gaz circulant dans le passage de gaz en fonction de la pression gazeuse s'exerçant dans le volume interne de l'élément de valve déformable.
- la collerette annulaire et l'élément de rondelle de l'élément de valve déformable sont agencés (i.e pris en sandwich) entre le corps de valve rigide et une surface du ventilateur, en particulier le fond du logement à valve.
- il comprend une carcasse externe comprenant un logement à valve débouchant vers l'extérieur et au sein duquel est agencée la valve expiratoire.
- le logement à valve comprend un fond où débouche la ligne d'amenée de pression, la collerette annulaire et l'élément de rondelle de l'élément de valve déformable étant positionnés en regard du fond du logement à valve.
- un joint d'étanchéité fluidique est agencé dans le fond du logement.
- le joint d'étanchéité fluidique est pris en sandwich entre l'élément de rondelle positionné sur la collerette annulaire de l'élément de valve déformable et le fond du logement à valve.
- au moins une partie du logement à valve a une forme tubulaire, typiquement cylindrique.
- le corps creux de l'élément de valve déformable comprend une paroi ou membrane souple, i.e. flexible, située à l'opposé de l'ouverture.
- la paroi ou membrane souple du corps creux de l'élément de valve déformable coopère avec un siège de valve porté par le corps de valve rigide.
- le siège de valve est aménagé dans le passage de gaz, de préférence dans la paroi du corps de valve rigide.
- le siège de valve a une forme circulaire, typiquement annulaire.
- le corps de valve rigide est en polymère.
- le circuit de gaz du ventilateur comprend en outre une branche inspiratoire alimentée par une source de gaz.
- la source gaz est agencée dans la carcasse du ventilateur.
- la source gaz comprend une micro-soufflante, aussi appelée turbine ou compresseur.
- la source gaz est pilotée par des moyens pilotage.
- les moyens pilotage comprennent au moins un microprocesseur, de préférence agencé sur une carte électronique.
- le microprocesseur est de type microcontrôleur.
- les moyens pilotage sont agencés dans la carcasse du ventilateur.
- le ventilateur comprend en outre un écran d'affichage, de préférence un écran numérique en couleurs ou en noir et blanc.
- le ventilateur comprend en outre des moyens de réglage ou de sélection, par exemple un ou plusieurs boutons rotatifs, touches, y compris virtuelles, de sélection ou autres.
- une source de courant électrique alimente électriquement les moyens pilotage et/ou la micro-soufflante et/ou l'écran d'affichage et/ou tout autre composant du ventilateur nécessitant de l'énergie électrique pour fonctionner (e.g. LED, etc...).
- la source de courant électrique comprend une batterie rechargeable ou une liaison secteur, ou les deux.
- la ligne d'amenée de pression est relié fluidiquement à la source de gaz.
- la ligne d'amenée de pression comprend un conduit ou passage de gaz ou analogue.

L'invention va maintenant être mieux comprise grâce à la description détaillée suivante, faite à titre illustratif mais non limitatif, en référence aux figures annexées parmi lesquelles :
Fig. 1 représente une vue de face d'un ventilateur médical selon l'invention,
Fig. 2 représente une vue latérale du ventilateur médical de Fig. 1,
Fig. 3 représente une vue du logement à valve expiratoire du ventilateur médical des Fig. 1 et Fig. 2,
Fig. 4 est une vue en coupe de la valve expiratoire agencée dans le ventilateur médical des Fig. 1 et Fig. 2,
Fig. 5 est une vue élargie d'une partie de la valve expiratoire de Fig. 4,
Fig. 6 est une vue en coupe de l'élément de valve déformable d'une valve expiratoire classique,
Fig. 7 est un mode de réalisation de l'élément de valve déformable d'une valve expiratoire selon l'invention,
Fig. 8 est une courbe de pression illustrant le phénomène de « frein expiratoire » pendant un cycle respiratoire du patient, se produisant dans un ventilateur médical selon l'art antérieur,
Fig. 9 est une courbe de pression comparative illustrant la suppression du phénomène de « frein expiratoire », pendant un cycle respiratoire du patient, obtenue en utilisant une valve expiratoire et un ventilateur selon l'invention, et
Fig. 10 schématise un mode de réalisation particulier de l'élément de valve déformable d'une valve expiratoire selon l'invention.

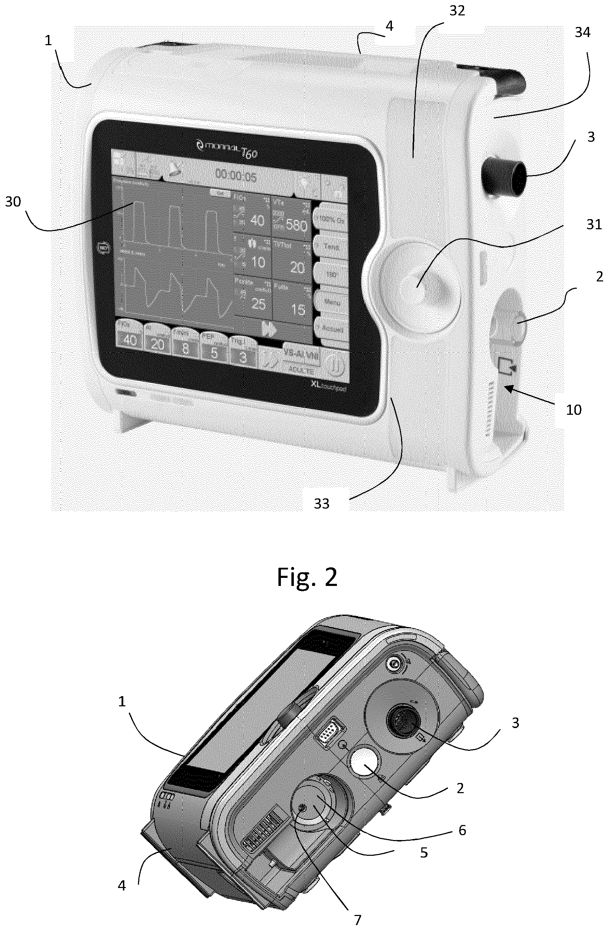

Fig. 1 et Fig. 2 représentent un ventilateur médical 1 selon l'invention, lequel comprend une carcasse 4 rigide externe, par exemple en polymère, portant, sur sa façade avant 33, un écran d'affichage 30, un bandeau lumineux 32 pouvant adopter un éclairage de plusieurs couleurs (e.g. rouge, vert...), fixes ou intermittentes, en fonction du fonctionnement du ventilateur 1, et un bouton rotatif 31 permettant d'opérer des sélections et/ou des validations, par exemple au sein de menus s'affichant sur l'écran d'affichage 30.

L'une face latérale 34 de la carcasse 4 du ventilateur médical 1 comprend des connecteurs en communication fluidique avec la branche inspiratoire 3 et la branche expiratoire 2, à savoir des conduits ou passages de gaz, du circuit de gaz interne agencé dans le ventilateur médical 1.

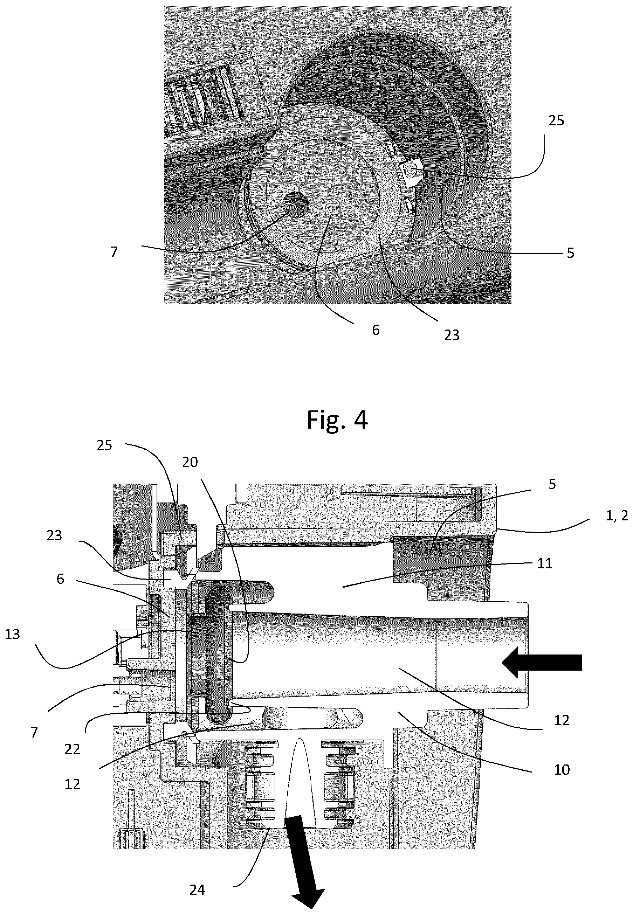

Il est prévu aussi un logement 5, agencé dans la face latérale 34, de la carcasse 4 au sein duquel vient se fixer la valve expiratoire 10, comme montré en Fig. 4. Comme mieux visible en Fig. 3, le logement 5 comprend un fond 6 percé d'un orifice 7 d'amenée de pression. Le logement 5 a une forme générale au moins partiellement cylindrique.

La valve expiratoire 10, montrée en Fig. 4, est formée d'un corps de valve 11 rigide, par exemple en polymère, traversé par un passage de gaz 12 dans lequel peut circuler le débit de gaz expiré.

Le corps de valve 11 rigide coopère avec un élément de valve 13 déformable agencé sur le passage de gaz 12, servant à contrôler la circulation de gaz dans le passage de gaz 12 du corps de valve 11.

Un joint d'étanchéité fluidique 23 est prévu dans le fond 6 du logement 5 afin de garantir une absence de fuites de la surpression amenée par la ligne d'amenée de pression 7, par exemple un joint torique ou analogue. Le joint d'étanchéité fluidique est agencé de sorte d'être pris en sandwich entre l'élément annulaire formant la collerette 17 de l'élément de valve déformable 13 et le fond 6 du logement à valve 5.

Par ailleurs, il est prévu un mécanisme de fixation 25, par exemple un doigt d'indexage, permettant de verrouiller mécaniquement la valve expiratoire 10 au sein du logement 5, comme illustré en Fig. 3. Bien entendu, tout autre mécanisme de fixation 25 adapté est utilisable.

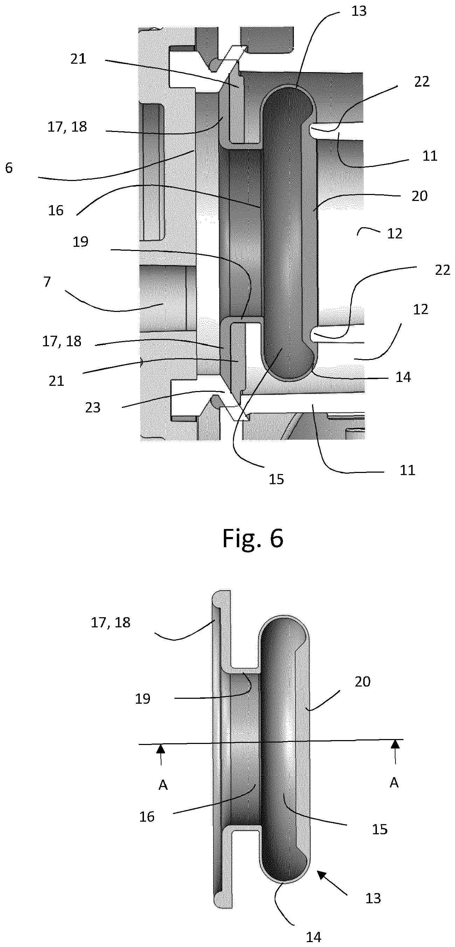

Comme illustré en Fig. 6, l'élément de valve 13 déformable est formé d'un corps creux 14 définissant un volume interne 15, constituant une chambre de pression, et comportant par ailleurs une ouverture 16, de préférence large, communiquant fluidiquement avec le volume interne 15, c'est-à-dire un passage de gaz. Une collerette annulaire 17 est solidarisée au corps creux 14 via un col 19 de forme tubulaire. La collerette annulaire 17 est agencé coaxialement (axe AA) autour de l'ouverture large 16 et plus précisément, autour du col 19 de forme tubulaire. Autrement dit, la collerette annulaire 17 forme un épaulement 18 annulaire se projetant en éloignement, i.e. radialement, par rapport à ouverture 16.

Le corps creux 14 de l'élément de valve 13 déformable comprend une paroi ou surface souple 20, i.e. flexible, située à l'opposé de l'ouverture large 16, venant coopérer avec un siège de valve 22 porté par le corps de valve 11 rigide. Le siège de valve 22 est agencé sur le passage de gaz 12. De préférence, le siège de valve 22 a une forme circulaire.

La paroi souple 20 constitue une membrane flexible pouvant se déformer sous l'effet de la pression du gaz s'exerçant dans le volume interne 15 du corps creux 14 de l'élément de valve déformable 13. En faisant varier la pression dans le volume interne 15 grâce à la surpression amenée par la ligne d'amenée de pression 7 débouchant au niveau du fond 6 du logement 5, la paroi souple 20 formant membrane flexible vient plus ou moins obturer le passage de gaz 12 du corps de valve rigide 11, en coopérant avec le siège de valve 22, ce qui permet de contrôler, i.e. fixer ou ajuster, la PEP et donc de contrôler l'échappement du gaz expiré par le patient (i.e. contenant du CO₂) vers l'atmosphère, via l'orifice d'échappement 24 de la valve 10 (cf. Fig. 4), pendant les phases expiratoires du patient. Pendant les phases d'inspiration, la membrane flexible 20 obture totalement le passage de gaz 12 du corps de valve rigide 11, en créant une étanchéité fluidique avec le siège de valve 22 du corps de valve rigide 11, et donc empêcher au gaz de circuler au travers de la valve 10 et d'être échappé vers l'atmosphère, via l'orifice d'échappement 24.

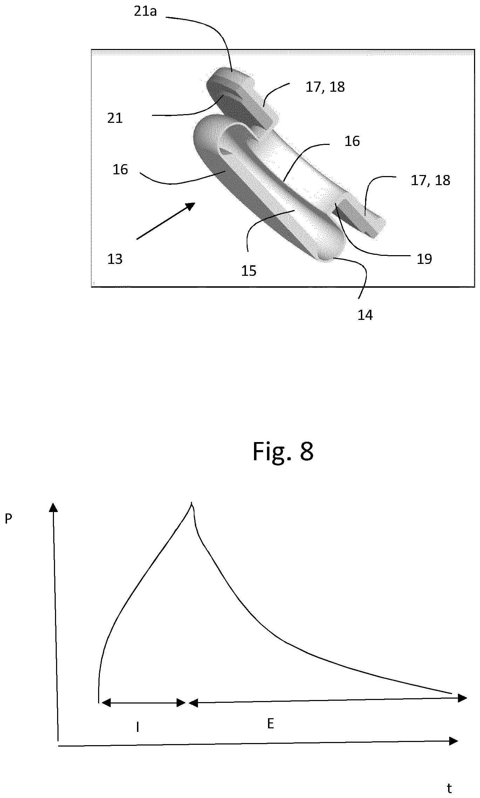

Selon l'invention, afin d'éviter le phénomène de « frein expiratoire » causé par un affaissement de tout ou partie de l'élément de valve 13 déformable, le corps creux 14 et la collerette annulaire 17, et préférentiellement le col 19 de forme tubulaire, sont formés, par exemple par moulage par injection ou autre, d'une pièce unique en un premier matériau souple, c'est-à-dire flexible, et un élément de rondelle 21 formé d'un second matériau de rigidité supérieure au premier matériau est agencé sur la collerette annulaire 17 formant épaulement. La Fig. 7 illustre ce mode de réalisation de l'élément de valve déformable 13 bi-matière d'une valve expiratoire 10 selon l'invention.

Avantageusement, l'élément de rondelle 21 est une rondelle rigide, c'est-à-dire un disque mince avec un trou, i.e. une ouverture traversante, en son centre. Elle vient se superposer à toute ou partie de la collerette annulaire 17 en étant agencée coaxialement à celle-ci et à l'ouverture 16 du corps creux 14.

Par exemple, le second matériau peut présenter une dureté Shore A différente, typiquement supérieure, à celle du premier matériau souple constituant le reste de l'élément de valve 13 déformable, à savoir le corps creux 14 et la collerette annulaire 17, et préférentiellement le col 19. Ainsi, le corps creux 14 et la collerette annulaire 17, et préférentiellement le col 19 peuvent être formés d'un premier matériau souple présentant une première dureté Shore A (dsA1), par exemple un élastomère de type silicone, alors que l'élément de rondelle 21 peut être formé d'un second matériau présentant une seconde dureté Shore A (dsA2) supérieure à la première dureté Shore A (dsA1). Par exemple, le premier matériau peut présenter une première dureté Shore A (dsA1) inférieure ou égale à 50 shore A, de préférence inférieure ou égale à environ 40 shore A, alors que le second matériau peut présenter une seconde dureté (dsA2) d'au moins 80 shore A.

Avantageusement, l'élément de rondelle 21 est une rondelle, i.e. une pièce annulaire plate ou analogue, recouvrant la collerette annulaire 17, c'est-à-dire s'étendant entre la base du col 19 auquel est attachée la collerette 17 et le rebord périphérique circulaire 21a de la collerette 17, comme illustré sur Fig. 7.

L'élément de rondelle 21 peut être soit simplement posé sur la collerette annulaire 17 sans y être fixé, soit y être fixé par exemple collé ou autre. La collerette annulaire 17 forme donc une base-support servant à recevoir la rondelle annulaire 21 de manière à la recouvrir totalement.

Selon un mode de réalisation préféré, l'élément de rondelle 21, e.g. la rondelle, est en un second matériau de type polymère, préférentiellement en polyamide (PA) ou polyacétal (POM, i.e. polyoxyméthylène), par exemple de type PA-66 ou POM-C, et par ailleurs, le corps creux 14, le col 19 et la collerette annuaire 17 sont formés d'une pièce en silicone, de préférence de type Silpuran^{®} 6000/40 A/B, en tant que premier matériau.

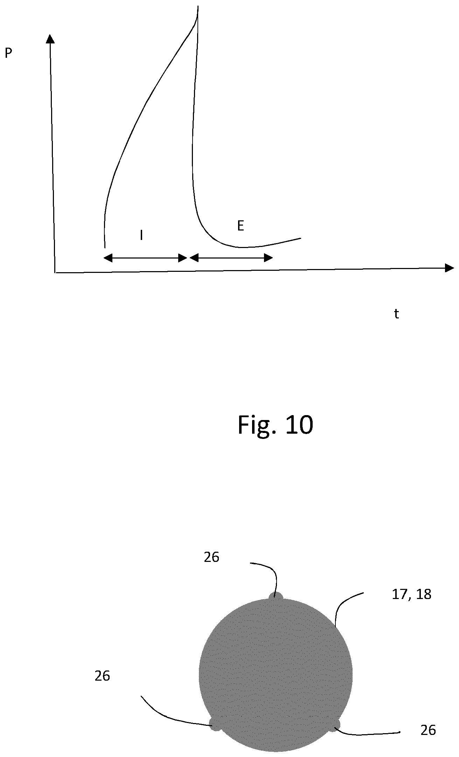

Un élément de valve 13 déformable comprenant une telle rondelle 21 en PA ou en POM et, par ailleurs, de tels corps creux 14, col 19 et collerette annuaire 17 en silicone de type Silpuran^{®} 6000/40 A/B, a montré de très bons résultats lors d'essais de fonctionnement d'un ventilateur selon l'invention équipé d'un tel élément de valve 13 au sein de sa valve expiratoire 10, en conduisant à l'obtention d'une PEP conforme à ce qui était souhaité avec évacuation aisée des gaz riches en CO₂ mais sans génération de « frein expiratoire » intempestif. Des résultats d'essais sont consignés sur les Fig. 8 et Fig. 9 et détaillés ci-après.

En choisissant avec soin les premier et second matériaux, on peut éviter, réduire ou minimiser le phénomène de « frein expiratoire » qui est une sollicitation mécanique de l'élément de valve déformable 13, qui induit une pression sur la collerette 17 se traduisant par un écrasement non-souhaité du corps creux 14 de l'élément de valve déformable 13.

En effet, utiliser un second matériau présentant une résistance supérieure, par exemple une seconde dureté (dsA2) d'au moins 80 shore A, pour réaliser l'élément de rondelle 21 permet de résoudre ce problème d'affaissement car l'élément de rondelle 21 va absorber la sollicitation mécanique. Il s'ensuit que le corps creux 14 ne reçoit pas de sollicitation mécanique et il n'y a pas d'effet d'écrasement.

Selon un mode de réalisation particulier, le premier matériau utilisé pour réaliser le corps creux 14, le col 19 et la collerette annuaire 17 est de la silicone dite « médicale » ou « biocompatible ».

Selon un autre mode de réalisation, la collerette annuaire 17 peut comprendre des "picots" 26 ou analogues, aussi appelés « godrons », permettant d'assurer une bonne insertion de l'élément de valve déformable 13 dans le corps de valve 11 de la valve expiratoire et un meilleur maintien/centrage de celui-ci. Ces picots 26 sont agencés sur la périphérie externe circulaire 21a de la collerette annuaire 17, de préférence angulairement répartis, e.g. 0°, 120°, 240°, et se projettent radialement vers l'extérieur, i.e. font saillie autour de la périphérie circulaire 21a, comme visible en Fig. 10. Toutefois, de tels picots 26 ne sont qu'optionnels.

Fig. 8 est une courbe de pression (P) illustrant le phénomène de « frein expiratoire » pendant un cycle respiratoire du patient (i.e. au fil du temps t), se produisant dans un ventilateur médical selon l'art antérieur, alors que Fig. 9 est une courbe de pression comparative illustrant la suppression de ce phénomène de « frein expiratoire », pendant le même cycle respiratoire du patient, obtenue en utilisant une valve expiratoire 11 et un ventilateur 1 selon l'invention. Le cycle respiratoire comprend une phase inspiratoire (I) suivie d'une phase expiratoire (E). La durée d'un tel cycle est de l'ordre de 3 à 6 secondes.

Comme on le voit sur Fig. 8, le phénomène de « frein expiratoire » se traduit sur par une lente diminution de la pression (P) pendant la phase expiratoire (E), alors que la diminution de pression (P) pendant la phase expiratoire (E) est beaucoup plus rapide sur Fig. 9. Ceci confirme l'efficacité de la solution de l'invention.

## Revendications

1. Ventilateur médical (1) comprenant un circuit de gaz (2, 3) avec une branche expiratoire (2) communiquant fluidiquement avec l'atmosphère et comprenant une valve expiratoire (10), ladite valve expiratoire (10) comprenant un corps de valve (11) rigide traversé par un passage de gaz (12) et un élément de valve (13) déformable agencé sur ledit passage de gaz (12) de manière à contrôler la circulation de gaz dans ledit passage de gaz (12), ledit élément de valve (13) déformable comprenant :
- un corps creux (14) définissant un volume interne (15) et comprenant une ouverture (16) communiquant avec ledit volume interne (15), et
- une collerette annulaire (17) solidaire du corps creux (14) et agencée coaxialement autour de ladite ouverture (16),
**caractérisé en ce que** :
- le corps creux (14) et la collerette annulaire (17) sont formés d'un premier matériau souple et
- un élément de rondelle (21) formé d'un second matériau de rigidité supérieure au premier matériau est agencé sur la collerette annulaire (17).

2. Ventilateur selon la revendication 1, **caractérisé en ce que** le premier matériau présente une première dureté Shore A (dsA1) inférieure ou égale à 50 shore A.

3. Ventilateur selon la revendication 1, **caractérisé en ce que** l'élément de rondelle (21) est une rondelle.

4. Ventilateur selon l'une des revendications 1 ou 3, **caractérisé en ce que** l'élément de rondelle (21) est en polymère.

5. Ventilateur selon la revendication 4, **caractérisé en ce que** le polymère est un polyamide (PA) ou un polyacétal (POM).

6. Ventilateur selon la revendication 1, **caractérisé en ce que** le premier matériau souple formant le corps creux (14) et la collerette annulaire (17) est un élastomère, de préférence en silicone.

7. Ventilateur selon l'une des revendications précédentes, **caractérisé en ce que** le corps creux (14) et la collerette annulaire (17) sont fixés l'un à l'autre par un col tubulaire (19) agencé coaxialement à l'ouverture (16) du corps creux (14).

8. Ventilateur selon l'une des revendications précédentes, **caractérisé en ce que** le corps creux (14) et la collerette annulaire (17) sont formés d'une seule pièce, de préférence par moulage par injection.

9. Ventilateur selon l'une des revendications précédentes, **caractérisé en ce que** l'élément de rondelle (21) repose sur la collerette annulaire (17) sans y être fixé.

10. Ventilateur selon la revendication 1, **caractérisé en ce que** la collerette annulaire (17) forme un épaulement (18) annulaire se projetant en éloignement par rapport à ouverture (16).

11. Ventilateur selon la revendication 1, **caractérisé en ce qu'**une ligne d'amenée de pression (7) est reliée fluidiquement à l'ouverture (16) du corps creux (14) de l'élément de valve (13) déformable de manière à fournir une pression gazeuse au volume interne (15) du corps creux (14).

12. Ventilateur selon la revendication 1, **caractérisé en ce que** le corps creux (14) définissant le volume interne (15) de l'élément de valve (13) déformable comprend une paroi flexible (20) venant au moins partiellement obturer le passage de gaz (12) du corps de valve (11) rigide de manière à contrôler ou ajuster le débit de gaz circulant dans le passage de gaz (12) en fonction de la pression gazeuse s'exerçant dans le volume interne (15) de l'élément de valve (13) déformable.

13. Ventilateur selon la revendication 1, **caractérisé en ce que** la collerette annulaire (17) et l'élément de rondelle (21) sont agencés entre le corps de valve (11) rigide et une surface (6) du ventilateur (1).

14. Ventilateur selon la revendication 1, **caractérisé en ce qu'**il comprend une carcasse externe (4) comprenant un logement à valve (5) débouchant vers l'extérieur et au sein duquel est agencée la valve expiratoire (10).

15. Ventilateur selon la revendication 14, **caractérisé en ce que** le logement à valve (5) comprend un fond (6) où débouche la ligne d'amenée de pression (7), la collerette annulaire (17) de l'élément de valve (13) déformable étant positionné en regard du fond (6) du logement à valve (5).

## Patentansprüche

1. Medizinisches Beatmungsgerät (1), welches einen Gaskreislauf (2, 3) mit einem Ausatmungszweig (2) umfasst, der mit der Atmosphäre in Fluidverbindung steht und ein Ausatemventil (10) umfasst, wobei das Ausatemventil (10) einen starren Ventilkörper (11), der von einem Gasdurchlass (12) durchquert wird, und ein verformbares Ventilelement (13), das so an dem Gasdurchlass (12) angeordnet ist, dass es die Gasströmung in dem Gasdurchlass (12) steuert, umfasst, wobei das verformbare Ventilelement (13) umfasst:
- einen Hohlkörper (14), der ein Innenvolumen (15) definiert und eine Öffnung (16) umfasst, die mit dem Innenvolumen (15) in Verbindung steht, und
- einen ringförmigen Kragen (17), der mit dem Hohlkörper (14) fest verbunden ist und koaxial um die Öffnung (16) herum angeordnet ist,
**dadurch gekennzeichnet, dass**:
- der Hohlkörper (14) und der ringförmige Kragen (17) aus einem flexiblen ersten Material ausgebildet sind und
- ein Rundscheibenelement (21), das aus einem zweiten Material mit einer Steifigkeit ausgebildet ist, die höher als die des ersten Materials ist, auf dem ringförmigen Kragen (17) angeordnet ist.

2. Beatmungsgerät nach Anspruch 1, **dadurch gekennzeichnet, dass** das erste Material eine erste Shore-Härte A (dsA1) aufweist, die kleiner oder gleich 50 Shore A ist.

3. Beatmungsgerät nach Anspruch 1, **dadurch gekennzeichnet, dass** Rundscheibenelement (21) eine runde Scheibe ist.

4. Beatmungsgerät nach einem der Ansprüche 1 oder 3, **dadurch gekennzeichnet, dass** das Rundscheibenelement (21) aus Polymer besteht.

5. Beatmungsgerät nach Anspruch 4, **dadurch gekennzeichnet, dass** das Polymer ein Polyamid (PA) oder ein Polyacetal (POM) ist.

6. Beatmungsgerät nach Anspruch 1, **dadurch gekennzeichnet, dass** das flexible erste Material, das den Hohlkörper (14) und den ringförmigen Kragen (17) bildet, ein Elastomer ist, vorzugsweise aus Silikon.

7. Beatmungsgerät nach einem der vorhergehenden Ansprüche, **dadurch gekennzeichnet, dass** der Hohlkörper (14) und der ringförmige Kragen (17) durch einen rohrförmigen Hals (19) aneinander befestigt sind, der koaxial mit der Öffnung (16) des Hohlkörpers (14) angeordnet ist.

8. Beatmungsgerät nach einem der vorhergehenden Ansprüche, **dadurch gekennzeichnet, dass** der Hohlkörper (14) und der ringförmige Kragen (17) aus einem Stück geformt sind, vorzugsweise durch Spritzgießen.

9. Beatmungsgerät nach einem der vorhergehenden Ansprüche, **dadurch gekennzeichnet, dass** das Rundscheibenelement (21) auf dem ringförmigen Kragen (17) ruht, ohne an ihm befestigt zu sein.

10. Beatmungsgerät nach Anspruch 1, **dadurch gekennzeichnet, dass** der ringförmige Kragen (17) einen ringförmigen Ansatz (18) bildet, der von der Öffnung (16) wegragt.

11. Beatmungsgerät nach Anspruch 1, **dadurch gekennzeichnet, dass** eine Druckzufuhrleitung (7) mit der Öffnung (16) des Hohlkörpers (14) des verformbaren Ventilelements (13) in Fluidverbindung steht, um dem Innenvolumen (15) des Hohlkörpers (14) einen Gasdruck zu liefern.

12. Beatmungsgerät nach Anspruch 1, **dadurch gekennzeichnet, dass** der Hohlkörper (14), der das Innenvolumen (15) des verformbaren Ventilelements (13) definiert, eine flexible Wand (20) umfasst, die den Gasdurchlass (12) des starren Ventilkörpers (11) wenigstens teilweise verschließt, um den Durchfluss von in dem Gasdurchlass (12) strömendem Gas in Abhängigkeit von dem Gasdruck, der in dem Innenvolumen (15) des verformbaren Ventilelements (13) ausgeübt wird, zu steuern oder anzupassen.

13. Beatmungsgerät nach Anspruch 1, **dadurch gekennzeichnet, dass** der ringförmige Kragen (17) und das Rundscheibenelement (21) zwischen dem starren Ventilkörper (11) und einer Fläche (6) des Beatmungsgeräts (1) angeordnet sind.

14. Beatmungsgerät nach Anspruch 1, **dadurch gekennzeichnet, dass** es ein äußeres Gehäuse (4) umfasst, das eine Ventilaufnahme (5) umfasst, die nach außen mündet und in deren Innerem das Ausatemventil (10) angeordnet ist.

15. Beatmungsgerät nach Anspruch 14, **dadurch gekennzeichnet, dass** die Ventilaufnahme (5) einen Boden (6) umfasst, wo die Druckzufuhrleitung (7) mündet, wobei der ringförmige Kragen (17) des verformbaren Ventilelements (13) gegenüber dem Boden (6) der Ventilaufnahme (5) positioniert ist.

## Claims

1. Medical ventilator (1) comprising a gas circuit (2, 3) with an expiratory branch (2) communicating fluidically with the atmosphere and comprising an expiratory valve (10), said expiratory valve (10) comprising a rigid valve body (11), through which a gas passage (12) extends, and a deformable valve element (13) arranged on said gas passage (12) in such a way as to control the flow of gas in said gas passage (12), said deformable valve element (13) comprising:
- a hollow body (14) defining an internal volume (15) and comprising an opening (16) communicating with said internal volume (15), and
- an annular flange (17) secured to the hollow body (14) and arranged coaxially around said opening (16),
**characterized in that**:
- the hollow body (14) and the annular flange (17) are formed from a first flexible material, and
- a washer element (21) formed from a second material of greater rigidity than the first material is arranged on the annular flange (17).

2. Ventilator according to Claim 1, **characterized in that** the first material has a first Shore A hardness (dsA1) of less than or equal to 50 Shore A.

3. Ventilator according to Claim 1, **characterized in that** the washer element (21) is a washer.

4. Ventilator according to either of Claims 1 and 3, **characterized in that** the washer element (21) is made of polymer.

5. Ventilator according to Claim 4, **characterized in that** the polymer is a polyamide (PA) or a polyacetal (POM).

6. Ventilator according to Claim 1, **characterized in that** the first flexible material forming the hollow body (14) and the annular flange (17) is an elastomer, preferably silicone.

7. Ventilator according to one of the preceding claims, **characterized in that** the hollow body (14) and the annular flange (17) are fixed to each other by a tubular neck (19) arranged coaxially with respect to the opening (16) of the hollow body (14).

8. Ventilator according to one of the preceding claims, **characterized in that** the hollow body (14) and the annular flange (17) are formed in one piece, preferably by injection moulding.

9. Ventilator according to one of the preceding claims, **characterized in that** the washer element (21) rests on the annular flange (17) without being fixed thereto.

10. Ventilator according to Claim 1, **characterized in that** the annular flange (17) forms an annular shoulder (18) projecting away from the opening (16).

11. Ventilator according to Claim 1, **characterized in that** a pressure supply line (7) is fluidically connected to the opening (16) of the hollow body (14) of the deformable valve element (13) in such a way as to supply a gas pressure to the internal volume (15) of the hollow body (14).

12. Ventilator according to Claim 1, **characterized in that** the hollow body (14) defining the internal volume (15) of the deformable valve element (13) comprises a flexible wall (20) which at least partially closes off the gas passage (12) of the rigid valve body (11) in such a way as to control or adjust the flow of gas circulating in the gas passage (12) according to the gas pressure exerted in the internal volume (15) of the deformable valve element (13).

13. Ventilator according to Claim 1, **characterized in that** the annular flange (17) and the washer element (21) are arranged between the rigid valve body (11) and a surface (6) of the ventilator (1).

14. Ventilator according to Claim 1, **characterized in that** it comprises an external casing (4) comprising a valve housing (5) which opens to the outside and within which the expiratory valve (10) is arranged.

15. Ventilator according to Claim 14, **characterized in that** the valve housing (5) comprises a bottom (6) where the pressure supply line (7) emerges, the annular flange (17) of the deformable valve element (13) being positioned opposite the bottom (6) of the valve housing (5) .
